# EUROPÄISCHE PATENTSCHRIFT

(11) **EP 2 593 492 B1**
(45) Veröffentlichungstag und Bekanntmachung des Hinweises auf die Patenterteilung: **20.08.2014**
(21) Anmeldenummer: 11738670.6
(22) Anmeldetag: 11.07.2011
(51) Int. Cl.: C08G 18/10, C08G 18/08, B29B 7/76, C07C 263/16

(54) **VERFAHREN ZUR HERSTELLUNG VON ISOCYANATGRUPPENHALTIGEN POLYURETHAN-PRÄPOLYMEREN**
PROCESS FOR PREPARING POLYURETHANE PREPOLYMERS CONTAINING ISOCYANATE GROUPS
PROCÉDÉ POUR PRÉPARER DES PRÉPOLYMÈRES DE POLYURÉTHANNE CONTENANT DES GROUPES ISOCYANATE

(30) Priorität: 13.07.2010 DE 102010027052
(43) Veröffentlichungstag der Anmeldung: 22.05.2013
(73) Patentinhaber: Bayer Intellectual Property GmbH, 40789 Monheim (DE)
(72) Erfinder: PIRKL, Hans-Georg, 51377 Leverkusen (DE); SCHMIDT, Manfred, 41540 Dormagen (DE); VIELER, Robert, 41542 Dormagen (DE); TRACHT, Ursula, 51379 Leverkusen (DE); WEUTA, Peter, 51375 Leverkusen (DE); BUCHHOLZ, Sigurd, 50767 Köln (DE)
(74) Vertreter: BIP Patents
(86) Internationale Anmeldenummer: PCT/EP2011/061739
(87) Internationale Veröffentlichungsnummer: WO 2012/007419

(56) Entgegenhaltungen:
- EP-A2- 0 480 588
- WO-A1-01/91897
- DE-A1- 19 823 393

## Beschreibung

Die vorliegende Erfindung betrifft das technische Gebiet der Isocyanate. Gegenstand der vorliegenden Erfindung ist ein Verfahren zur Herstellung von NCO-Präpolymeren durch Umsetzen eines stöchiometrischen Überschusses eines organischen Isocyanats mit einer isocyanatreaktiven Komponente, wobei die Reaktionskomponenten vermischt werden und die Mischung unmittelbar in einen Lagerungs- oder Transportbehälter gefüllt wird, wo sie vollständig abreagiert.

Isocyanatgruppenhaltige Polyurethan-Präpolymere, die durch Umsetzung eines stöchiometrischen Überschusses eines organischen Polyisocyanats mit einem organischen Polyol erhalten werden können, sind im Bereich der Polyurethane bekannt. Sie weisen einen Gehalt an freiem Isocyanat von 2-15 Gew.-% auf und werden beispielsweise bei der Herstellung von Elastomeren, Beschichtungen, Haftmitteln und dergleichen verwendet.

Die Herstellung solcher NCO-Präpolymere ist in zahlreichen Schriften beschrieben worden.

In DE69132613T2 ist beschrieben, dass die Reaktion zwischen einem Polyol und einem Poylisocyanat exotherm verläuft. Um eine vollständige Umsetzung in akzeptabler Zeit zu erreichen, würde das Reaktionsgemisch normalerweise auf eine Temperatur im Bereich von etwa 40 bis etwa 100°C für eine Stunde oder länger erhitzt.

In WO94/29361A1 wird die Herstellung von Präpolymeren mit NCO-Gehalten von 5 bis 10 Gew.-% durch Umsetzung von Isocyanaten mit Polyolen in einem Temperaturbereich von 40 bis 80°C beschrieben, wobei die Umsetzung konventionell, d.h. in einem dafür geeigneten Reaktionsgefäß erfolgt.

Gemäß DE2823762A1 erfolgt die Reaktion zwischen Diisocyanaten und Polyhydroxyverbindungen zur Synthese von thermoplastischen Polyurethanen dadurch, dass die Ausgangskomponenten durch eine Mischzone und danach durch eine Reaktionszone geleitet werden. Laut Beschreibung der DE2823762A1 verbleiben die Reaktionskomponenten solange in der Reaktionszone, dass die Polyaddition vollständig erfolgt.

DE19823392A1 beschreibt eine Umsetzung von Isocyanat mit Polyol im Prozessstrom der isocyanaterzeugenden Anlage bei Temperaturen von 40 bis 100°C. In EP0722962A2 wird eine Schaumanlage genutzt, insbesondere deren Mischkopf, um sowohl Präpolymere herzustellen als auch um diese anschließend zu verschäumen.

Die im Stand der Technik beschriebenen Verfahren zur Herstellung von NCO-Präpolymeren bedürfen einer Umsetzung einer isocyanatgruppenhaltigen Komponente mit einer isocyanatgruppenreaktiven Komponente in einem Reaktor und insbesondere unter kontrollierten Misch- und Temperaturbedingungen.

Ausgehend vom Stand der Technik stellt sich einem Fachmann die Aufgabe, ein flexibles Verfahren zur Herstellung von NCO-Präpolymeren bereitzustellen, mit dem NCO-Präpolymere auf einfache und schnelle Weise erzeugt werden können. Es wäre wünschenswert, wenn die Herstellung von NCO-Präpolymere auch möglich wäre, wenn keine Produktionsanlagen verfügbar sind. Ferner wäre es wünschenswert, wenn es möglich wäre, verschiedene NCO-Präpolymere zu erzeugen, ohne dass es hoher Aufwände z.B. in Bezug auf die Reinigung von Reaktionsanlagen bedarf.

Überraschend wurde gefunden, dass eine isocyanatgruppenhaltige Komponente im stöchiometrischen Überschuss und eine isocyanatgruppenreaktive Komponente im stöchiometrischen Unterschuss bei Raumtemperatur in einer Mischvorrichtung kontinuierlich zusammengeführt werden können und die reaktive Mischung unmittelbar in einen Lagerungs- oder Transportbehälter gegeben werden kann, wo die Komponenten ohne weitere Durchmischung und ohne Temperaturkontrolle reproduzierbar zu einem isocyanatgruppenhaltigen Präpolymer reagieren.

Ein erster Gegenstand der vorliegenden Erfindung ist daher ein Verfahren zur Herstellung eines isocyanatgruppenhaltigen Präpolymers aus einer isocyanatgruppenhaltigen Komponente und einer isocyanatgruppenreaktiven Komponente, dadurch gekennzeichnet, dass die Komponenten kontinuierlichen in einer Mischvorrichtung vermischt und das Reaktionsgemisch unmittelbar nach dem Mischen kontinuierlich in einen Lagerungs- oder Transportbehälter gefüllt wird, wo die Umsetzung zwischen den Komponenten vollendet wird.

Im Unterschied zu den im Stand der Technik beschriebenen Verfahren erfolgt die Umsetzung einer isocyanatgruppenhaltigen Komponente und einer isocyanatgruppenreaktiven Komponente nicht in einem konventionellen Reaktionsgefäß sondern die Komponenten werden in einer Mischvorrichtung kontinuierlich zusammengeführt und die reagierende Mischung wird direkt in einen Lagerungsbehälter oder einen Transportbehälter gefüllt. Gegenstand der vorliegenden Erfindung ist insofern auch ein Verfahren zum Abfüllen einer reagierenden Mischung umfassend eine isocyanatgruppenhaltige und eine isocyanatgruppenreaktive Komponente in einen Lagerungs- oder Transportbehälter, wobei das Abfüllverfahren dieselben Merkmale umfasst wie das erfindungsgemäße Verfahren zur Herstellung eines isocyanatgruppenhaltigen Präpolymers. Im Folgenden wird daher nicht weiter zwischen dem Abfüllverfahren und dem Herstellverfahren unterschieden. Wenn das Abfüllverfahren oder das Herstellverfahren näher erläutert wird, sind stets beide Verfahren gemeint.

Im Folgenden wird für die Begriffe Lagerungsbehälter und Transportbehälter auch der allgemeine Begriff Behälter verwendet.

Die Zusammenführung der Komponenten und die Abfüllung der reaktiven Mischung in den Behälter erfolgen üblicherweise kontinuierlich solange bis der Behälter gefüllt ist. Dann wird in der Regel der Prozess der Zusammenführung und Abfüllung gestoppt und gegebenenfalls zu einem späteren Zeitpunkt fortgeführt, wenn ein noch nicht gefüllter Behälter vorliegt. Insofern werden die Prozesse der Zusammenführung der Komponenten und die Abfüllung der reaktiven Mischung absatzweise kontinuierlich durchgeführt.

Die Umsetzung zwischen den Komponenten findet hauptsächlich in dem Behälter statt. Das bedeutet, dass die Verweilzeit in der Mischvorrichtung im Wesentlichen nur der Vermischung der Komponenten dient. Der Behälter stellt einen Raum zur Verfügung, in dem die Komponenten miteinander reagieren und das gewünschte Produkt bilden können.

In einer bevorzugten Ausführungsform der vorliegenden Erfindung ist der durch den Behälter bereitgestellte Reaktionsraum transportabel, d.h. er dient dem Transport des Produkts zu seinem Bestimmungsort. Das erfindungsgemäße Vorgehen hat damit den Vorteil, dass keine Produktionskapazitäten zur Herstellung von isocyanatgruppenhaltigen Präpolymeren bereitgestellt werden müssen. Die Reaktionskomponenten können je nach Bedarf aus Lagertanks direkt über eine Mischvorrichtung in einen Transportbehälter überführt werden. Somit ermöglicht die Erfindung den absatzweisen Betrieb zur schnellen Befüllung einzelner Produktgebinde ohne zusätzliche Reaktionsräume oder Puffertanks. Die Umsetzung der Reaktionskomponenten zum gewünschten Produkt kann vollzogen werden, während sich der Transportbehälter bereits auf dem Weg zum Bestimmungsort befindet.

Unter einem Transportbehälter wird dabei ein Behälter verstanden, der üblicherweise dem Transport von Chemikalien, insbesondere von Isocyanaten dient. Der Behälter ist transportabel ausgeführt; das heißt, er kann mit einem geeigneten Transportmittel an einen anderen Ort bewegt werden.

Insbesondere ist ein Transportbehälter dadurch gekennzeichnet, dass er in der Regel keine Mittel zur Durchmischung und/oder keine Mittel zur Temperaturkontrolle aufweist. Auf derartige Mittel, wie insbesondere Mittel zur Kühlung, kann bei dem erfindungsgemäßen Verfahren verzichtet werden.

In einer weiteren bevorzugten Ausführungsform ist der Behälter ein Lagerbehälter, in dem das Reaktionsprodukt gelagert werden kann.

Vorzugsweise werden die Reaktionskomponenten so zusammengeführt, dass die Temperaturerhöhung in dem verwendeten Behälter geringer ist als 60°C; bevorzugt ist die Temperaturerhöhung geringer als 40°C, besonders bevorzugt geringer als 20°C, noch bevorzugter geringer als 10°C und am meisten bevorzugt maximal 5°C. Eine geringe Temperaturerhöhung ist bevorzugt, da der Behälter keine Mittel zur Kühlung umfasst und daher aus sicherheitstechnischen Gründen die Temperaturerhöhung zu begrenzen ist. Zudem kann eine Temperaturerhöhung negativen Einfluss auf die Produktqualität ausüben.

Vorzugsweise wird die oben genannte maximale Temperaturerhöhung durch das Mischungsverhältnis der Reaktionskomponenten eingestellt.

Bei dem erfindungsgemäßen Verfahren werden eine isocyanatgruppenhaltige Komponente im stöchiometrischen Überschuss und eine isocyanatgruppenreaktive Komponente im stöchiometrischen Unterschuss miteinander umgesetzt. Bevorzugt beträgt der Anteil an isocyanatgruppenreaktiver Komponente in der Mischung 0,1 bis 10 Gew.-%, besonders bevorzugt 0,5 bis 8 Gew.-%, ganz besonders bevorzugt 1 bis 5 Gew.-%.

Durch die vorliegende Erfindung können ohne Reinigung von Reaktionsanlagen auf einfache und schnelle Weise neue Produkte dadurch erzeugt werden, dass das Mischverhältnis der Komponenten und/oder die Einsatzstoffe variiert werden und so unterschiedliche Präpolymere mit einem variablen Isocyanatgehalt resultieren.

Die Reaktionskomponenten, d.h. die isocyanatgruppenhaltige und die isocyanatgruppenreaktive Komponente werden über eine Mischvorrichtung zusammengeführt bevor die reagierende Mischung in den Behälter gelangt. Die Zusammenführung der Komponenten erfolgt durch geeignete Fördermittel wie beispielsweise Pumpen aus getrennten Vorlagen. Beispielsweise beträgt die Summe der Volumenströme der Komponenten bei der Abfüllung der reaktiven Mischung in einen Tankwagen mit einem Fassungsvermögen von 20 Tausend Litern und einer tolerierten Betankungszeit von 60 Minuten etwa 5,6 Liter/sec.

Die erforderliche Verweilzeit in der Mischvorrichtung hängt von der Mischwirkung der Mischvorrichtung ab. Ziel der Vermischung ist die Erzeugung einer "homogenen" Verteilung der Komponenten in der reagierenden Mischung. Dabei ist eine "homogene" Verteilung im Sinne der vorliegenden Erfindung dann erreicht, wenn eine weitergehende auf die Verteilung ausgeübte Mischwirkung zu keiner merklichen Änderung des resultierenden Produkts hinsichtlich seiner chemischen und physikalischen Eigenschaften führt.

Überraschend wurde gefunden, dass Mischzeiten von wenigen Sekunden ausreichend sind.

Die Mischvorrichtung weist einen oder mehrere statische und/oder dynamische Mischer auf. Als dynamische Mischer kommen beispielsweise Rotor/Stator-Homogenisierer (wie Megatron FM10/50/2 der Fa. Kinematica, Schweiz) oder vergleichbare Geräte in Frage.

Vorzugsweise werden statische Mischer verwendet. Während bei dynamischen Mischern die Homogenisierung einer Mischung durch bewegte Organe erreicht wird, wird bei statischen Mischern die Strömungsenergie des Fluids ausgenutzt: eine Fördereinheit (z.B. eine Pumpe) drückt das Fluid durch ein mit statischen Mischereinbauten versehenes Rohr, wobei das der Hauptströmungsachse folgende Fluid in Teilströme aufgeteilt wird, die je nach Art der Einbauten gedehnt, geschert, miteinander verwirbelt und vermischt werden.

Einen Überblick über verschiedene Typen von statischen Mischern gibt der Artikel "Statische Mischer und ihre Anwendungen", M. H. Pahl und E. Muschelknautz, Chem.-Ing.-Techn. 52 (1980) Nr. 4, S. 285-291.
Erfindungsgemäß einsetzbare Statikmischer sind beispielsweise auch in Chem.-Ing. Techn. 52, Nr. 4, Seite 285 bis 291 sowie in "Mischen von Kunststoff und Kautschukprodukten", VDI-Verlag, Düsseldorf 1993, beschrieben. Bevorzugt werden Mischer mit gekreuzten Stegen eingesetzt, wie sie in DE2532355A1 beschrieben werden. Beispielhaft seien SMX-Statikmischer der Firma Sulzer genannt. Es ist auch denkbar, mikrostrukturierte Statikmischer einzusetzen wie sie beispielsweise von der Fa. Ehrfeld Mikrotechnik BTS GmbH vertrieben werden; diese neigen jedoch eher zur Verblockung.

Entscheidend für das Verhindern von Verstopfungen vor, hinter und/oder in der Mischvorrichtung während der Stillstände zwischen zwei Abfüllvorgängen sind Spülzyklen mit einer Komponente in Verbindung mit einem geeigneten Ventil für den Zulauf der anderen Komponente.

In einer bevorzugten Ausführungsform wird in einem ersten Schritt zunächst nur eine erste Reaktionskomponente, vorzugsweise die isocyanatgruppenhaltige Komponente, durch die Mischvorrichtung in den Behälter geleitet. Dieser Vorlauf ist Bestandteil des Produktes. In einem zweiten Schritt wird zusätzlich zur ersten Reaktionskomponente die zweite Reaktionskomponente, vorzugsweise die isycyanatgruppenreaktive Komponente ebenfalls in die Mischvorrichtung geleitet, wo sie mit der ersten Reaktionskomponente vermischt wird. Mit anderen Worten werden während des zweiten Schrittes sowohl die erste als auch die zweite Reaktionskomponente in die Mischvorrichtung geleitet. Die Mischung gelangt unmittelbar in den Behälter. Zum Beenden der Lot-Produktion wird in einem dritten Schritt die Dosierung der zweiten Reaktionskomponente gestoppt und die Mischvorrichtung mit der ersten Reaktionskomponente gespült. Hierbei kommt der Spülung der Dosiereinheit für die zweite Reaktionskomponente besondere Bedeutung zu: der unmittelbar vor der Mischvorrichtung befindliche Teil des Zulauf für die zweite Reaktionskomponente wird beispielsweise über einen Dreiwegehahn zunächst mit einer Inertsubstanz, vorzugsweise Stickstoff oder ein Edelgas, und erst dann mit der ersten Reaktionskomponente gespült. Anschließend verbleibt die Mischvorrichtung im gefluteten Zustand (mit der ersten Reaktionskomponente geflutet) bei Umgebungstemperatur bis zum Start der nächsten Lot-Produktion.

Für das erfindungsgemäße Verfahren kommen als isocyanatgruppenhaltige Komponente beispielsweise die an sich bekannten aliphatischen, cycloaliphatischen, araliphatischen und aromatischen mehrwertigen Isocyanate in Frage.

Beispielhaft seien folgende Isocyanate genannt: Alkylendiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylendiisocyanat-1,5, Tetramethylendiisocyanat-1,4 und vorzugsweise Hexamethylendiisocyanat-1,6, cycloaliphatische Diisocyanate, wie Cyclohexan-1,3-und -1,4-diisocyanat sowie beliebige Gemische dieser Isomeren, 1-Isocyanato-3,3,5-trimethyl-5-isocyanatomethylcyclohexan (IPDI), 2,4- und 2,6-Hexahydrotoluylendiisocyanat sowie die entsprechenden Isomerengemische, 4,4'-, 2,2'- und 2,4'-Dicyclohexlylmethandiisocyanat sowie die entsprechenden Isomerengemische, und vorzugsweise aromatische Di- und Polyisocyanate, wie z. B. 2,4- und 2,6-TDI und die entsprechenden Isomerengemische, 4,4'-, 2,4'- und 2,2'-MDI und die entsprechenden Isomerengemische, Mischungen aus 4,4'- und 2,2'-MDI, Polyphenylpolymethylenpolyisocyanate, Mischungen aus 4,4'-, 2,4'- und 2,2'-MDI und Polyphenylpolymethylenpolyisocyanaten (Roh-MDI) und Mischungen aus Roh-MDI und TDI. Die organischen Di- und Polyisocyanate können einzeln oder in Form beliebiger Mischungen eingesetzt werden.

Als isocyanatgruppenreaktive Komponenten kommen Verbindungen oder Mischungen von Verbindungen in Frage, die über ein oder mehrere azide Wasserstoffatome verfügen.

Als Verbindungen mit mindestens zwei aziden Wasserstoffatomen werden zweckmäßigerweise solche mit einer Funktionalität von 2 bis 4, vorzugsweise 2 bis 3, und einem Molekulargewicht von 300 bis 8000, vorzugsweise von 300 bis 5000, verwendet. Bewährt haben sich z.B. Polyetherpolyamine und/oder vorzugsweise Polyole ausgewählt aus der Gruppe der Polyetherpolyole, Polyesterpolyole, Polythioetherpolyole, Polyesteramide, hydroxylgruppenhaltigen Polyacetale und hydroxylgruppenhaltigen aliphatischen Polycarbonate oder Mischungen aus mindestens zwei der genannten Polyole. Vorzugsweise Anwendung finden Polyesterpolyole und/oder Polyetherpolyole. Die Hydroxylzahl der Polyhydroxylverbindungen beträgt dabei in aller Regel 20 bis 80 und vorzugsweise 28 bis 56 mg KOH/g.

Geeignete Polyesterpolyole können beispielsweise aus organischen Dicarbonsäuren mit 2 bis 12 Kohlenstoffatomen, vorzugsweise aliphatischen Dicarbonsäuren mit 4 bis 6 Kohlenstoffatomen, und mehrwertigen Alkoholen, vorzugsweise Diolen, mit 2 bis 12, vorzugsweise 2 bis 6, Kohlenstoffatomen, hergestellt werden. Als Dicarbonsäuren kommen beispielsweise in Betracht: Bernsteinsäure, Glutarsäure, Adipinsäure, Korksäure, Azelainsäure, Sebacinsäure, Decandicarbonsäure, Maleinsäure, Fumarsäure, Phthalsäure, Isophthalsäure und Terephthalsäure. Die Dicarbonsäuren können dabei sowohl einzeln als auch im Gemisch untereinander verwendet werden. Anstelle der freien Dicarbonsäuren können auch die entsprechenden Dicarbonsäurederivate, wie z. B. Dicarbonsäureester von Alkoholen mit 1 bis 4 Kohlenstoffatomen oder Dicarbonsäureanhydride, eingesetzt werden. Vorzugsweise verwendet werden Dicarbonsäuregemische aus Bernstein-, Glutar- und Adipinsäure in Mengenverhältnissen von beispielsweise 20 bis 35 : 35 bis 50 : 20 bis 32 Gew.-Teilen und insbesondere Adipinsäure. Beispiele für zwei- und mehrwertige Alkohole, insbesondere Diole, sind: Ethandiol, Diethylenglykol, 1,2- bzw. 1,3-Propandiol, Dipropylenglykol, 1,4-Butandiol, 1,5-Pentandiol, 1,6-Hexandiol, 1,10-Decandiol, Glycerin und Trimethylolpropan. Vorzugsweise verwendet werden Ethandiol, Diethylenglykol, 1,4-Butandiol, 1,5-Pentandiol und 1,6-Hexandiol. Eingesetzt werden koennen ferner Polyesterpolyole aus Lactonen, z. B. ε-Caprolacton oder Hydroxycarbonsaeuren, z. B. ω-Hydroxycapronsaeure.

Zur Herstellung der Polyesterpolyole können die organischen, z. B. aromatischen und vorzugsweise aliphatischen, Polycarbonsäuren und/oder -derivate und mehrwertigen Alkohole katalysatorfrei oder vorzugsweise in Gegenwart von Veresterungskatalysatoren, zweckmäßigerweise in einer Atmosphäre aus Inertgas, wie z. B. Stickstoff, Kohlenmonoxid, Helium, Argon u. a., in der Schmelze bei Temperaturen von 150 bis 250°C, vorzugsweise 180 bis 220°C, gegebenenfalls unter vermindertem Druck bis zu der gewünschten Säurezahl, die vorteilhafterweise kleiner als 10, vorzugsweise kleiner als 2, ist, polykondensiert werden. Nach einer bevorzugten Ausführungsform wird das Veresterungsgemisch bei den oben genannten Temperaturen bis zu einer Säurezahl von 80 bis 30, vorzugsweise 40 bis 30, mg KOH/g unter Normaldruck und anschließend unter einem Druck von kleiner als 500 mbar, vorzugsweise 50 bis 150 mbar, polykondensiert. Als Veresterungskatalysatoren kommen beispielsweise Eisen-, Cadmium-, Kobalt-, Blei-, Zink-, Antimon-, Magnesium-, Titan- und Zinnkatalysatoren in Form von Metallen, Metalloxiden oder Metallsalzen in Betracht. Die Polykondensation kann jedoch auch in flüssiger Phase in Gegenwart von Verdünnungs- und/oder Schleppmitteln, wie z. B. Benzol, Toluol, Xylol oder Chlorbenzol, zur azeotropen Abdestillation des Kondensationswassers durchgeführt werden.

Zur Herstellung der Polyesterpolyole werden die organischen Polycarbonsäuren und/oder -derivate und mehrwertigen Alkohole vorteilhafterweise im Molverhältnis von 1:1 bis 1,8, vorzugsweise 1:1,05 bis 1,2, polykondensiert.

Die erhaltenen Polyesterpolyole besitzen vorzugsweise eine Funktionalität von 2 bis 4, insbesondere 2 bis 3, und ein Molekulargewicht von 480 bis 3000, insbesondere 600 bis 2000.

Insbesondere als Polyole verwendet werden jedoch Polyetherpolyole, die nach bekannten Verfahren, beispielsweise durch anionische Polymerisation mit Alkalihydroxiden, wie z. B. Natrium- oder Kaliumhydroxid, oder Alkalialkoholaten, wie z. B. Natriummethylat, Natrium- oder Kaliumethylat oder Kaliumisopropylat, als Katalysatoren und unter Zusatz mindestens eines Startermoleküls, das 2 bis 4, vorzugsweise 2 bis 3, reaktive Wasserstoffatome gebunden enthält, oder durch kationische Polymerisation mit Lewissäuren, wie Antimonpentachlorid, Borfluorid-Etherat u. a., oder Bleicherde, als Katalysatoren aus einem oder mehreren Alkylenoxiden mit 2 bis 4 Kohlenstoffatomen im Alkylenrest hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Als Startermoleküle kommen beispielsweise in Betracht: Wasser, organische Dicarbonsäuren, wie Bernsteinsäure, Adipinsäure, Phthalsäure und Terephthalsäure, aliphatische und aromatische, gegebenenfalls N-mono-, N,N-und N,N'-dialkylsubstituierte Diamine mit 1 bis 4 Kohlenstoffatomen im Alkylrest, wie gegebenenfalls mono- und dialkylsubstituiertes Ethylendiamin, Diethylentriamin, Triethylentetramin, 1,3-Propylendiamin, 1,3- bzw. 1,4-Butylendiamin, 1,2-, 1,3-, 1,4-, 1,5- und 1,6-Hexamethylendiamin, Phenylendiamin, 2,3-, 2,4- und 2,6-Toluylendiamin, und 4,4'-, 2,4'- und 2,2'-Diaminodiphenylmethan. Als Startermolekuele kommen ferner in Betracht: Alkanolamine, wie z. B. Ethanolamin, N-Methyl- und N-Ethylethanolamin, Dialkanolamine, wie z. B. Diethanolamin, N-Methyl- und N-Ethyldiethanolamin, und Trialkanolamine, wie z. B. Triethanolamin, und Ammoniak. vorzugsweise verwendet werden mehrwertige, insbesondere zwei- und/oder dreiwertige Alkohole, wie Ethandiol, Propandiol-1,2 und -2,3, Diethylenglykol, Dipropylenglykol, Butandiol-1,4, Hexandiol-1,6, Glycerin, Trimethylolpropan und Pentaerythrit, während für Hartschaumpolyetherole vorwiegend höherfunktionelle Starter, wie z. B. Sorbit und Saccharose, zur Anwendung kommen.

Die Polyetherpolyole, vorzugsweise Polyoxypropylen- und Polyoxypropylenpolyoxyethylenpolyole, besitzen eine Funktionalität von vorzugsweise 2 bis 4 und insbesondere 2 bis 3 und Molekulargewichte von 300 bis 8000, vorzugsweise 300 bis 6000 und insbesondere 1000 bis 5000, und geeignete Polyoxytetramethylenglykole ein Molekulargewicht bis ungefähr 3500, während bei Hartschaumpolyetherolen Molekulargewichte von 300 bis 1000 die Regel sind.

Als Polyetherpolyole eignen sich ferner polymermodifizierte Polyetherpolyole, vorzugsweise Pfropfpolyetherpolyole, insbesondere solche auf Styrol- und/oder Acrylnitrilbasis, die durch *in situ* Polymerisation von Acrylnitril, Styrol oder vorzugsweise Mischungen aus Styrol und Acrylnitril, z. B. im Gewichtsverhältnis 90 : 10 bis 10 : 90, vorzugsweise 70 : 30 bis 30 : 70, in zweckmäßigerweise den vorgenannten Polyetherpolyolen analog den Angaben in DE 11 11 394, DE 12 22 669 (US-A-3,304,273, US-A-3,383,351, US-A-3,523,093), DE 11 52 536 (GB 1040452) und DE 11 52 537 (GB 987618) hergestellt werden, sowie Polyetherpolyoldispersionen, die als disperse Phase, üblicherweise in einer Menge von 1 bis 50 Gew.-%, vorzugsweise 2 bis 25 Gew.-%, enthalten: z. B. Polyharnstoffe, Polyhydrazide, tertiäre Aminogruppen gebunden enthaltende Polyurethane und/oder Melamin und die z. B. beschrieben werden in der EP-B-011 752 (US-A-4,304,708), US-A-4,374,209 und DE-A-32 31 497.

Die Polyetherpolyole können ebenso wie die Polyesterpolyole einzeln oder in Form von Mischungen verwendet werden. Ferner können sie mit den Pfropfpolyetherpolyolen oder Polyesterpolyolen sowie den hydroxylgruppenhaltigen Polyesteramiden, Polyacetalen, Polycarbonaten und/oder Polyetherpolyaminen gemischt werden.

Als hydroxylgruppenhaltige Polyacetale kommen z. B. die aus Glykolen, wie Diethylenglykol, Triethylenglykol, 4,4'-Dihydroxyethoxydiphenyldimethylmethan, Hexandiol und Formaldehyl herstellbaren Verbindungen in Frage. Auch durch Polymerisation cyclischer Acetale lassen sich geeignete Polyacetale herstellen.

Als Hydroxylgruppen aufweisende Polycarbonate kommen solche der an sich bekannten Art in Betracht, die beispielsweise durch Umsetzung von Diolen, wie Propandiol-1,3, Butandiol-1,4 und/oder Hexandiol-1,6, Diethylenglykol, Triethylenglykol oder Tetraethylenglykol mit Diarylcarbonaten, z. B. Diphenylcarbonat, oder Phosgen hergestellt werden können.

Zu den Polyesteramiden zählen z. B. die aus mehrwertigen, gesättigten und/oder ungesättigten Carbonsäuren bzw. deren Anhydriden und mehrwertigen gesättigten und/oder ungesättigten Aminoalkoholen oder Mischungen aus mehrwertigen Alkoholen und Aminoalkoholen und/oder Polyaminen gewonnenen, vorwiegend linearen Kondensate.

Geeignete Polyetherpolyamine können aus den oben genannten Polyetherpolyolen nach bekannten Verfahren hergestellt werden. Beispielhaft genannt seien die Cyanoalkylierung von Polyoxyalkylenpolyolen und anschließende Hydrierung des gebildeten Nitrils (US-A-3,267,050) oder die teilweise oder vollständige Aminierung von Polyoxyalkylenpolyolen mit Aminen oder Ammoniak in Gegenwart von Wasserstoff und Katalysatoren (DE 12 15 373).

Weiterhin können der isocyanatgruppenhaltige Komponente und/oder die isocyanatgruppenreaktive Komponente die dem Fachmann bekannten und geeignet erscheinenden Lösemittel, Hilfs- und/oder Zusatzstoffe und/oder Katalysatoren beigegeben werden (siehe z.B. DE 198 23 392 A1).

Die erfindungsgemäß hergestellten, schwach modifizierten Isocyanate eignen sich u.a. zur Herstellung von Polyurethanschäumen.

Die Erfindung wird nachstehend anhand von Beispielen näher erläutert, ohne sie jedoch hierauf zu beschränken.

Es zeigen:

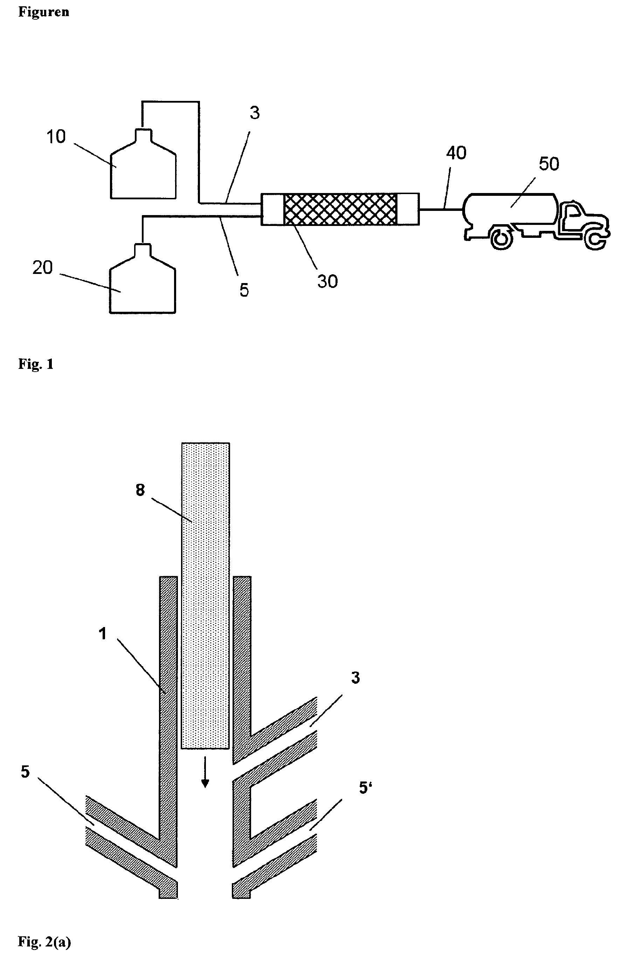

**Fig. 1** zeigt schematisch das erfindungsgemäße Konzept zum Abfüllen einer reagierenden Mischung umfassend eine isocyanatgruppenhaltige und eine isocyanatgruppenreaktive Komponente in einen Transportbehälter. Die Kompenten werden aus getrennten Vorlagen (10, 20) über die Zuführungen (3, 5) in eine Mischkammer 30 gegeben, in der die Komponenten homogen vermischt werden. Über einen Ablauf 40 gelangt die Mischung in den Tank 50 eines Tankwagens.

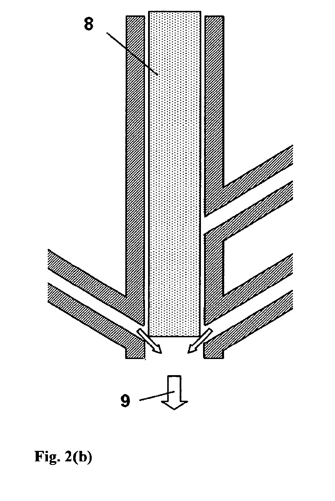

**Fig. 2(a)** und **Fig. 2(b)** zeigen schematisch einen Teil einer Vorrichtung zum Abfüllen einer reagierenden Mischung in einen Behälter. Der gezeigte Teil umfasst ein Rohr 1, in dem ein Kolben 8 eingebracht ist. Über Zuführungen 3, 5, 5' können reagierende Substanzen in das Rohr eingebracht werden. Dabei wird eine Komponente über die Zuführung 3 eingebracht und die andere Komponente über die Zuführungen 5 und 5'. Der Kolben 8 ist innerhalb des Rohres 1 beweglich ausgeführt.

Die Vorrichtung eignet sich insbesondere zum Zusammenführen einer isocyanatgruppenhaltigen Komponente und eine isocyanatgruppenreaktiven Komponente. Eine der Komponenten (K2) wird über die Zuführung 3 zugeführt; die andere Komponente (K1) über die Zuführungen 5 und 5'. Bei Beendigung der Zuführung wird der Kolben 8 nach unten bewegt. Er schließt dabei die Zuführung 3, so dass keine Komponente K2 mehr in das Rohr 1 gelangt. Bei der Bewegung nach unten drückt er Reste der Komponente K2 aus dem Rohr 1 in Richtung Mischkammer (die Richtung zur Mischkammer wird durch den Pfeil 9 angezeigt). Die Zuführungen 5 und 5' sind so angebracht, dass sie das untere Ende des Kolbens 8 mit der Komponente K1 abspülen, so dass keine Reste von K2 mehr in dem Rohr verbleiben. Die Zuführungen für die Komponenten K1 sind vorzugsweise in das Rohr 1 radial in Richtung auf den Kolben 8 angeordnet. Fig. 2(a) und Fig. 2(b) zeigen im Querschnitt 2 Zuführungen für die Komponente K1 (5, 5'). In einer bevorzugten Ausführungsform sind n Zuführungen für die Komponente K1 radial angeordnet, wobei n eine ganze Zahl im Bereich von 3 bis 20, bevorzugt im Bereich 3 bis 10, ganz bevorzugt im Bereich 4 bis 8 ist.

Die in den Figuren 2(a) und 2(b) gezeigte Vorrichtung ist so gestaltet, dass bei der erfindungsgemäßen Befüllung eines Behälters keine Reste / Strähnen an uneingemischter Komponente K2 in der Vorrichtung verbleiben. Reste dieser nicht reagierten bzw. nicht eingemischten Bulk-Phase würden ansonsten in der Vorrichtung weiterreagieren und ggf. zur Ausbildung von Verstopfungen führen.

### Bezugszeichen:

- 1: Rohr
- 3: Zuführung
- 5: Zuführung
- 5': Zuführung
- 8: Kolben
- 9: Richtung zur Mischkammer
- 10: Vorlagebehälter
- 20: Vorlagebehälter
- 30: Mischkammer
- 40: Ablauf
- 50: Transportbehälter

### Beispiele

### Kontinuierliche Umsetzung einer Polyol-Komponente mit einer Isocyanat-Komponente

Eine Polyol-Komponente und eine Isocyanat-Komponente wurden in einer Vorrichtung wie schematisch in den Figuren 2(a), (b) dargestellt zusammengeführt.

Die Polyol-Komponente wurde durch Umpumpen mittels Zahnradpumpe über einen ölbeheizten Wärmetauscher und durch Beheizen des Vorlagebehälters (10) auf die gewünschte Temperatur vorgewärmt. Für die Isocyanat-Komponente bestand ebenfalls die Möglichkeit einer Temperierung, alternativ wurde diese mit Umgebungstemperatur dosiert. Beide Edukte wurden über Zahnradpumpen einem Mischmodul (30) zugeführt.

Als Mischmodul diente in einem Fall ein statischer Mischer, Typ SMX der Firma Sulzer/CH, in einem anderen Fall ein dynamischer Mischer, Typ Megatron FM 10-50/2, Fa. Kinematica / CH. Das verwendete Mischmodul hatte jedoch keinen merklichen Einfluss auf die Produktqualität.

Die exakten Dosierströme beider Edukte wurden über Massedurchflussmesser erfasst und für die Flussratenregelung zugrunde gelegt.

Zum Anfahren einer Lot-Produktion wurde zunächst für kurze Zeit reines MDI durch die Anlage geleitet. Dieser Vorlauf war Bestandteil des Produktes und wurde im Produktbehälter aufgefangen. Der Hauptlauf begann mit dem Start der Polyol-Dosierung. Die gewünschte Zusammensetzung der reagierenden Mischung wurde sichergestellt, indem die Polyol-Dosierung während des Hauptlaufs entsprechend erhöht wurde, so dass die dosierten Gesamtmengen beider Komponenten dem Zielverhältnis Isocyanat:Polyol entsprachen. Hierbei wurde ebenfalls der während des Abfahrens entstehende Isocyanat-Nachlauf berücksichtigt. Die Mengen von Vorlauf und Nachlauf waren klein gegenüber der im Hauptlauf produzierten Mischungsmenge. Zum Beenden der Lot-Produktion wurde die Dosierung der Poylol-Komponente gestoppt und die Anlage mit reinem Isocyanat durchgespült. Anschließend verblieb die Anlage im gefluteten Zustand bei Umgebungstemperatur bis zum Start der nächsten Lot-Produktion. Diese Verfahrensweise hatte den zusätzlichen Vorteil, dass keine Abfälle entstehen.

Tabelle 1 fasst verschiedene Verfahrensläufe und ihre Ergebnisse zusammen. Alle Edukte sind kommerziell bei der Firma Bayer MaterialScience AG erhältlich.

Es bedeuten:

| | |
|---|---|
| MDI 44V20L: | Desmodur® 44 V 20 L, Gemisch von 4,4'-Diphenylmethandiisocyanat (MDI) und höherfunktionellen Homologen (pMDI) mit einer Viskosität bei 25 °C von ≥ 160 mPas bis ≥ 240 mPas; Bayer MaterialScience AG. Das Produkt enthält ca. 45% 2-Kern MDI. |
| PEP 53D: | Polyesterpolyol auf der Basis von Adipinsäure, Phthalsäure und Diethylenglykol mit einer OH-Zahl von ca. 210 mg(KOH) / g und einer Viskosität von 10,4 Pa s bei 20 °C; Bayer MaterialScience AG. |
| P293: | Bifunktionelles Polyesteretherpolyol, EO-Addukt an eine Mischung aus Phthalsäureanhydrid, Diethylenglycol und Ethylendiamin mit einer OH-Zahl von 275 bis 325 mg(KOH) / g und einer Viskosität von 6,5 +/- 1,3 Pa s bei 25 °C; Bayer MaterialScience AG. |
| DEG: | Diethylenglykol, OH-Zahl 1055 mg(KOH) / g, Viskosität 38 mPa s bei 20 °C. |

**Tabelle 1**

| **Isocyanat-Komponente** | **Polyol-Komponente** | **Gewichtsanteil Polyol integral** | **Massenstrom Isocyanat [kg/h]** | **Massenstrom Polyol [kg/h]** | **Vorlauf + Nachlauf [%]** | **Temperatur Isocyanat [°C]** | **Temperatur Polyol [°C]** | **Viskosität [mPas]** | **NCO-Gehalt [%]** |
|---|---|---|---|---|---|---|---|---|---|
| MDI 44V20L | PEP 53D | 5.0 | 427 | 23 | 0 | 40 | 40 | 590 | 28.8 |
| MDI 44V20L | P293 | 4.0 | 384 | 16 | 0 | 40 | 40 | 724 | 29.0 |
| MDI 44V20L | P293 | 4.0 | 384 | 17.7 | 33 | 40 | 40 | 729 | 29.0 |
| MDI 44V20L | P293 | 3.5 | 386 | 14.5 | 4.3 | 40 | 40 | 635 | 29.0 |
| MDI 44V20L | P293 | 3.5 | 386 | 14.5 | 4.3 | 40 | 40 | 540 | 29.3 |
| MDI 44V20L | P293 | 3.5 | 347.4 | 12.6 | 0 | 17 | 40 | 550 | 29.4 |
| MDI 44V20L | DEG | 1.5 | 394 | 6 | 0 | 40 | 20 | 660 | 29.2 |

## Patentansprüche

1. Verfahren zur Herstellung eines isocyanatgruppenhaltigen Präpolymers aus einer isocyanatgruppenhaltigen Komponente und einer isocyanatgruppenreaktiven Komponente, **dadurch gekennzeichnet, dass** die Komponenten kontinuierlich in einer Mischvorrichtung vermischt werden und das Reaktionsgemisch unmittelbar nach dem Mischen kontinuierlich in einen Lagerungs- oder Transportbehälter gefüllt wird, wo die Umsetzung zwischen den Komponenten vollendet wird,
wobei in einem ersten Schritt einer Lot-Produktion zunächst nur isocyanatgruppenhaltige Komponente durch die Mischvorrichtung in den Behälter geleitet wird, bevor in einem zweiten Schritt die isocyanatgruppenreaktive Komponente zusätzlich zur isocyanatgruppenhaltigen Komponente ebenfalls in die Mischvorrichtung geleitet wird, wo sie mit der ersten Reaktionskomponente vermischt wird.
und wobei zum Beenden der Lot-Produktion die Dosierung der isocyanatruppenreaktiven Komponente gestoppt und die Mischvorrichtung mit der isocyanatgruppenhaltigen Komponente gespült wird.

2. Verfahren nach Anspruch 1, wobei der Lagerungs- oder Transportbehälter keine Mittel zur Durchmischung und/oder Kühlung aufweist.

3. Verfahren nach Anspruch 1 oder 2, **dadurch gekennzeichnet, dass** der Transportbehälter der Tank eines Tankwagens ist.

4. Verfahren nach einem der Ansprüche 1 bis 3, **dadurch gekennzeichnet, dass** die Temperaturerhöhung in dem Behälter geringer als 60°C ist.

5. Verfahren nach einem der Ansprüche 1 bis 4, **dadurch gekennzeichnet, dass** der Anteil an isocyanatgruppenreaktiver Komponente in der Mischung 0,1 bis 10 Gew.% beträgt.

6. Verfahren nach einem der Ansprüche 1 bis 5, **dadurch gekennzeichnet, dass** die Mischvorrichtung einen oder mehrere statische Mischer umfasst.

7. Verfahren nach Anspruch 1, wobei nach Beendigung der Lot-Produktion die Mischvorrichtung im gefluteten Zustand bei Umgebungstemperatur bis zum Start der nächsten Lot-Produktion verbleibt.

8. Verfahren zum Abfüllen einer reagierenden Mischung in einen Lagerungs- oder Transportbehälter, umfassend die Merkmale mindestens eines der Ansprüche 1 bis 7.

## Claims

1. Method for preparing an isocyanate group-containing prepolymer from an isocyanate group-containing component and an isocyanate group-reactive component, **characterised in that** the components are continuously mixed in a mixing device and immediately after being mixed the reaction mixture is introduced continuously into a storage or transport vessel, where the reaction between the components is completed, wherein in a first step of a batch production only an isocyanate group-containing component is initially directed through the mixing device into the vessel, before in a second step the isocyanate group-reactive component is likewise directed into the mixing device in addition to the isocyanate group-containing component, where it is mixed with the first reaction component, and wherein to end the batch production the metering of the isocyanate group-reactive component is stopped and the mixing device is rinsed with the isocyanate group-containing component.

2. Method according to claim 1, wherein the storage or transport vessel has no means of mixing and/or cooling.

3. Method according to claim 1 or 2, **characterised in that** the transport vessel is the tank of a tanker vehicle.

4. Method according to one of claims 1 to 3, **characterised in that** the rise in temperature in the vessel is less than 60°C.

5. Method according to one of claims 1 to 4, **characterised in that** the proportion of isocyanate group-reactive component in the mixture is 0.1 to 10 wt.%.

6. Method according to one of claims 1 to 5, **characterised in that** the mixing device comprises one or more static mixers.

7. Method according to claim 1, wherein after the end of the batch production the mixing device remains in the flooded state at ambient temperature until the start of the next batch production.

8. Method of introducing a reacting mixture into a storage or transport vessel, comprising the features of at least one of claims 1 to 7.

## Revendications

1. Procédé de fabrication d'un prépolymère contenant des groupes isocyanate à partir d'un composant contenant des groupes isocyanate et d'un composant réactif avec les groupes isocyanate, **caractérisé en ce que** les composants sont mélangés en continu dans un dispositif de mélange et le mélange réactionnel est transféré en continu immédiatement après le mélange dans un contenant de stockage ou de transport, où la réaction entre les composants est terminée,
lors d'une première étape de la production d'un lot, tout d'abord uniquement le composant contenant des groupes isocyanate étant introduit dans le contenant par le biais du dispositif de mélange, avant l'introduction lors d'une seconde étape du composant réactif avec les groupes isocyanate en plus du composant contenant des groupes isocyanate également dans le dispositif de mélange, où il est mélangé avec le premier composant réactionnel,
et, pour finir la production du lot, le dosage du composant réactif avec les groupes isocyanate étant arrêté et le dispositif de mélange étant rincé avec le composant contenant des groupes isocyanate.

2. Procédé selon la revendication 1, dans lequel le contenant de stockage ou de transport ne comprend pas de moyen de mélange et/ou de refroidissement.

3. Procédé selon la revendication 1 ou 2, **caractérisé en ce que** le contenant de transport est la citerne d'un camion-citerne.

4. Procédé selon l'une quelconque des revendications 1 à 3, **caractérisé en ce que** l'élévation de température dans le contenant est inférieure à 60 °C.

5. Procédé selon l'une quelconque des revendications 1 à 4, **caractérisé en ce que** la proportion de composant réactif avec les groupes isocyanate dans le mélange est de 0,1 à 10 % en poids.

6. Procédé selon l'une quelconque des revendications 1 à 5, **caractérisé en ce que** le dispositif de mélange comprend un ou plusieurs mélangeurs statiques.

7. Procédé selon la revendication 1, dans lequel, après la fin de la production d'un lot, le dispositif de mélange reste à l'état inondé à température ambiante jusqu'au début de la production du lot suivant.

8. Procédé de remplissage d'un contenant de stockage ou de transport avec un mélange en réaction, comprenant les caractéristiques selon au moins l'une quelconque des revendications 1 à 7.
